# EUROPÄISCHE PATENTSCHRIFT

(11) **EP 2 926 760 B1**
(45) Veröffentlichungstag und Bekanntmachung des Hinweises auf die Patenterteilung: **21.08.2019**
(21) Anmeldenummer: 15000991.8
(22) Anmeldetag: 29.06.2011
(51) Int. Cl.: A61K 6/00, A61C 5/00, A61K 6/02, A61K 6/083, A61C 13/087, A61C 5/20, A61C 5/70, A61C 5/77

(54) **ZAHNFRONTVERBLENDUNGSKÖRPER**
TOOTH FRONT VENEER
CORPS DE FACETTE AVANT DE DENT

(30) Priorität: 02.07.2010 AT 11242010
(43) Veröffentlichungstag der Anmeldung: 07.10.2015
(62) Teilanmeldung aus: 11743945.5
(73) Patentinhaber: Coltène/Whaledent AG, 9450 Altstätten (CH)
(72) Erfinder: Lampl, Stephan, 9450 Lüchingen (CH)
(74) Vertreter: Hepp Wenger Ryffel AG

(56) Entgegenhaltungen:
- DE-A1- 10 234 994
- DE-A1- 19 654 055
- US-A1- 2004 096 805

## Beschreibung

Die vorliegende Erfindung betrifft einen Zahnfrontverblendungskörper.

Zahnfrontverblendungskörper sind beim Stand der Technik bekannt. Sie werden häufig auch als Veneer bezeichnet. Es handelt sich um meist schalenförmig ausgebildete Festkörper, welche als Verblendung auf gegebenenfalls vorher abgeschliffene Zähne bzw. Zahnstümpfe aufgesetzt werden, um dem verfärbten Zahn wieder die gewünschte Optik zu geben. Sie ersetzen in der Regel somit die natürliche Frontfläche des jeweiligen Zahns.

Beim Stand der Technik werden diese Zahnfrontverblendungskörper aus Keramik gefertigt. Hierzu wird zunächst am abgeschliffenen Zahn bzw. Zahnstumpf z.B. mittels Abdruck die benötigte Geometrie des Zahnfrontverblendungskörpers bestimmt. Nach diesen Angaben wird dann der keramische Zahnfrontverblendungskörper im Labor gefertigt. In einem dritten Schritt muss dann der Zahnarzt diesen Zahnfrontverblendungskörper am Zahn bzw. Zahnstumpf anbringen. Wurde der keramische Zahnfrontverblendungskörper nicht mit der notwendigen Sorgfalt gefertigt, so muss ein neuer Zahnfrontverblendungskörper angefertigt werden, da eine Anpassung an die Gegebenheiten im Mund des Patienten vor Ort beim Zahnarzt nicht möglich ist. Dies bedeutet in Summe, dass der Patient zumindest zweimal zum Zahnarzt gehen muss, bis der passende Zahnfrontverblendungskörper hergestellt und am Zahn angebracht ist. Weiters bedeutet die Notwendigkeit der Laborarbeit einen erheblichen Zeit- und Kostenaufwand.

DE 102 34 994 A1 beschreibt eine Vorrichtung zur Anbringung einer Verblendung au feinen Zahn, welche nach dem Anbringen auf dem Zahn ausgehärtet wird.

US 2004/0096805 A1 beschreibt eine Prothese für eine Zahnoberfläche.

DE 196 54 055 beschreibt ein Halbzeug als Formkörper zur Herstellung von Zahnersatzteilen, u.a. von Veneers, durch Materialabtragen, wobei der Formkörper aus einer Basis und einer diese Basis zumindest teilweise bedeckende Schicht aufgebaut ist. Die Basis und die sie bedeckende Schicht sind aus unterschiedlich pigmentierten Dentalmaterialien aufgebaut, um das äussere Erscheinungsbild des natürlichen Zahnes zu ersetzen.

Aufgabe der Erfindung ist es, Zahnfrontverblendungskörper zur Verfügung zu stellen, welche bei einem einzigen Zahnarztbesuch in ihrer Grösse angepasst und am schadhaften Zahn bzw. Zahnstumpf angebracht werden können.

Erfindungsgemäß wird dies mittels eines Zahnfrontverblendungskörpers gemäß Anspruch 1 erreicht.

Ein wesentlicher Grundgedanke der Erfindung besteht damit darin, die Zahnfrontverblendungskörper nicht mehr aus keramischen Werkstoffen sondern aus einem Composite bzw. zusammengesetzten Material herzustellen, wobei das zusammengesetzte Material zumindest ein organisches Bindemittel und die genannten Feststoffpartikel als Füllstoff aufweist. Zahnfrontverblendungskörper dieser Art können in ihrer Form vor Ort vom Zahnarzt mit den in seiner Praxis üblichen Werkzeugen bearbeitet und damit an die beim Patient vorhandenen Vorgaben angepasst werden. Es ist somit nicht mehr notwendig, die Zahnfrontverblendungskörper im Labor herstellen zu lassen. Wie beim Stand der Technik handelt es sich bei den erfindungsgemäßen Zahnfrontverblendungskörpern bzw. Veneers um Festkörper, welche auf einen vorab wie beim Stand der Technik bekannt bearbeiteten Zahnstumpf bzw. Zahn aufgesetzt werden können, um die Frontfläche eines verfärbten Zahnes entsprechend zu verblenden und optisch aufzuwerten. Vor allem auf Grund der als Füllstoff verwendeten Feststoffpartikel weist der erfindungsgemäße Zahnfrontverblendungskörper aber auch die notwendige Festigkeit bzw. Härte auf, damit der mit dem Zahnfrontverblendungskörper versehene Zahn dauerhaft seiner eigentlichen Aufgabe als Kauwerkzeug nachkommen kann.

Günstig ist es in diesem Zusammenhang insbesondere, wenn die Feststoffpartikel des Füllstoffs Glas, vorzugsweise eine Mischung verschiedener Glasarten, aufweisen oder daraus bestehen. Die Glasarten können sich sowohl in ihrer Färbung als auch in ihrer Zusammensetzung unterscheiden. Z.B. ist es denkbar Bariumgläser oder Strontiumgläser oder Gemische daraus zu verwenden. Allgemein kann es sich um oberflächenbehandeltes SiO2 handeln. Um eine hohe Festigkeit des Zahnfrontverblendungskörpers zu erreichen, ist es günstig, die Feststoffpartikel des Füllstoffes in einer möglichst dichten Packung im Zahnfrontverblendungskörper anzuordnen. Hierzu ist es günstig, wenn der Füllstoff Feststoffpartikel mit voneinander verschiedenen Korngrößen aufweist. Generell gesprochen sehen bevorzugte Ausgestaltungsformen vor, dass die Korngrößen der Feststoffpartikel des Füllstoffs in einem Bereich zwischen 0,01 µm und 50 µm liegen. Um eine möglichst hohe Packungsdichte zu erreichen, sind aber insbesondere auch die kleinen Korngrößen wichtig, da sie die Zwischenräume zwischen den größeren Körnern füllen. In diesem Sinne ist es günstig, wenn der Füllstoff Feststoffpartikel mit Korngrößen zwischen 0,01 µm und 3 µm aufweist. Im Sinne der Festigkeit ist weiters ein möglichst hoher Füllgrad des zusammengesetzten Materials des Zahnfrontverblendungskörpers anzustreben. Der Anteil der Feststoffpartikel des Füllstoffs am zusammengesetzten Material sollte also möglichst hoch sein. Günstige Varianten der Erfindung sehen in diesem Zusammenhang vor, dass die Feststoffpartikel des Füllstoffs einen Anteil von zumindest 75 Vol-%, vorzugsweise von zumindest 82 Vol-%, am zusammengesetzten Material des Zahnfrontverblendungskörpers haben.

Als organische Bindemittel verwenden bevorzugte Ausgestaltungsformen der Erfindung Methacrylat basierte Bindemittel. Diese können aus Methacrylat bestehen oder Methacrylat, vorzugsweise als Hauptbestandteil, aufweisen.

Bei den Zahnfrontverblendungskörpern handelt es sich um Festkörper, welche bevorzugt zumindest bereichsweise schalenförmig gewölbt ausgebildet sind.

Eine wichtige Aufgabe beim Anbringen von Zahnfrontverblendungskörpern an Zähnen bzw. Zahnstümpfen ist es, die Farbgebung des mit dem Zahnfrontverblendungskörpers verblendeten Zahns so auszubilden, dass sie an die Gegebenheiten im Mund des Patienten, also auch an die benachbarten Zähne angepasst ist. Bevorzugte Ausgestaltungsformen der Erfindung sehen vor, dass der Zahnfrontverblendungskörper standardisiert eine Farbe aufweist, welche der Farbe von natürlichem Zahnschmelz nahekommt. Besonders bevorzugt ist in diesem Sinne vorgesehen, dass der Zahnfrontverblendungskörper transluzent bzw. durchscheinend, also nicht opak ausgebildet ist. Eine Idee besteht darin, die Farbanpassung vorzunehmen, indem man ein jeweils entsprechend eingefärbtes Befestigungsmaterial verwendet, welches beim Anbringen des Zahnfrontverblendungskörpers zwischen diesem und dem Zahn bzw. Zahnstumpf angeordnet ist und durch den transluzenten Zahnfrontverblendungskörper hindurchscheint, und so die endgültige, nach außen hin wahrnehmbare Farbe des Gesamtaufbaus prägt. Untersuchungen haben ergeben, dass der Zahnfrontverblendungskörper günstigerweise eine Transluzenz von 28% bis 39% und/oder einen L-Wert von 59 bis 69 und/oder einen a-Wert von -0,35 bis -3,2 und/oder einen b-Wert von -0,4 bis -6,95 aufweist. Bei der Transluzenz handelt es sich um die Lichtdurchlässigkeit, also die reziproke Eigenschaft zur Opazität. Die genannten Werte beziehen sich auf eine Messung gemäß DIN 6174. Auch der Lab-Farbraum ist beim Stand der Technik bekannt. Er ist auf Grundlage der Gegenfarbentheorie konstruiert und ermöglicht die farbmetrische Bestimmung von Farbmaßzahlen und Farbabständen im angenähert gleichförmigen CIELAB-Farbenraum. Die Bestimmung des L-Werts, des a-Werts und des b-Werts erfolgt ebenfalls gemäß den Vorgaben von DIN 6174. In Anwendung der Gegenfarbentheorie liegen sich hier Grün und Rot auf der a-Achse gegenüber. Die b-Achse entspricht den Gegenfarben Blau und Gelb. Die L-Achse steht auf dieser Ebene senkrecht und gibt die Helligkeit wieder.

Aufgrund ihrer hohen Festigkeit können die Zahnfrontverblendungskörper relativ dünn ausgeführt sein. Besonders bevorzugt ist dabei vorgesehen, dass der Zahnfrontverblendungskörper an einem seiner Endbereiche eine Schneidkante, vorzugsweise mit einer Dicke von 1,0mm bis 1,3mm, aufweist, wobei vorzugsweise vorgesehen ist, dass der Zahnfrontverblendungskörper außerhalb des Endbereichs mit der Schneidkante maximal eine Dicke von 0,6mm aufweist und/oder an seinem der Schneidkante gegenüberliegenden Ende flach ausläuft. Unter Dicke ist dabei jeweils die Wandstärke im entsprechenden Bereich des Zahnfrontverblendungskörpers zu verstehen.

Durch die erfindungsgemäßen Zahnfrontverblendungskörper wird es, wie gesagt, möglich, dass der Zahnarzt den Zahnfrontverblendungskörper bzw. einen entsprechenden Rohling vor Ort an die geometrischen Anforderungen des Zahns bzw. Zahnstumpfs des Patienten anpasst. Hat der Zahnarzt zusätzlich noch die Möglichkeit ein entsprechendes Befestigungsmaterial nach seiner Farbe auszusuchen, so kann er auch zusätzlich noch die gewünschte Farbanpassung vor Ort vornehmen. Um dem Zahnarzt, abgesehen von seiner normalen Ausstattung, alle für die Behandlung benötigten Hilfsmittel zur Verfügung zu stellen, sieht ein besonderer Aspekt der Erfindung ein Set mit mehreren, insbesondere verschieden großen und/oder verschieden geformten, erfindungsgemäßen Zahnfrontverblendungskörpern vor, wobei das Set zusätzlich zumindest ein, vorzugsweise flüssiges oder pastöses, Befestigungsmaterial und mehrere verschiedene Farben zum Einfärben des Befestigungsmaterials und/oder mehrere verschiedenfarbige, vorzugsweise flüssige oder pastöse, Befestigungsmaterialien aufweist, wobei das Befestigungsmaterial bzw. die Befestigungsmaterialien zum Befestigen, vorzugsweise Ankleben, des Zahnfrontverblendungskörpers an einem Zahn bzw. Zahnstumpf geeignet ist bzw. sind. Dies ermöglicht es, den Zahnarzt aus den ihm vom Set zur Verfügung gestellten Zahnfrontverblendungskörpern diejenige Größe auszusuchen, welche der natürlichen Situation am zu behandelnden Zahn oder Zahnstumpf am nächsten kommt. Die restliche Anpassung kann dann durch Bearbeiten bzw. Zuschleifen dieses Rohlings vor Ort vorgenommen werden. Hierzu kann der Zahnarzt auf die ihm standardmäßig zur Verfügung stehenden Schleifgeräte zurückgreifen. Weiters kann der Zahnarzt die geeignete Farbe bzw. das geeignet eingefärbte Befestigungsmaterial aussuchen, mit dem der Zahnfrontverblendungskörper am Zahn-stumpf bzw. Zahn befestigt, vorzugsweise angeklebt, wird, sodass auch die gewünschte Färbung des dann am Zahn bzw. Zahnstumpf befestigten Zahnfrontverblendungskörper erreicht ist. Um letztendlich verschiedenfarbiges Befestigungsmaterial zur Verfügung zu stellen, kann das Set in einer Ausgestaltungsform zumindest ein Befestigungsmaterial und mehrere verschiedene Farben aufweisen, mit denen das Befestigungsmaterial vom Zahnarzt eingefärbt wird. Bevorzugt ist jedoch vorgesehen, dass das Set bereits mehrere verschiedenfarbige Befestigungsmaterialien aufweist, bei welchen die Farbe nicht mehr vom Zahnarzt in das Befestigungsmaterial eingemischt werden muss. Das Befestigungsmaterial kann pastös, also breiig bzw. dickflüssig ausgebildet sein. Um kleinere Fissuren bearbeiten zu können, ist es aber auch denkbar, flüssiges Befestigungsmaterial im Set zur Verfügung zu stellen. Besonders bevorzugte Ausgestaltungsformen eines erfindungsgemäßen Sets sehen vor, dass das, vorzugsweise in pastöser Form vorliegende, Befestigungsmaterial dasselbe zusammengesetzte Material enthält wie die Zahnfrontverblendungskörper des Sets, allerdings eben noch in pastöser Konsistenz. Weiters ist es günstig, wenn das, insbesondere in flüssiger Form vorliegende, Befestigungsmaterial dasselbe organische Binde-mittel wie die Zahnfrontverblendungskörper des Sets, eben allerdings noch in flüssiger Form enthält.

Bevorzugte Ausgestaltungsformen des erfindungsgemäßen Sets sehen darüber hinaus vor, dass das Set zumindest einen Haftvermittler, vorzugsweise zumindest zwei verschieden Haftvermittler, und/oder zumindest ein Ätzmittel aufweist. Ein Haftvermittler bzw. das Bonding dienen dazu, die Verbindung zwischen dem hydrophilen Zahn und dem hydrophoben zusammengesetzen Material des Zahnfrontverblendungskörpers bzw. des Befestigungsmaterials herzustellen. Dabei sollte der Haftvermittler auch die Schrumpfkräfte des Befestigungsmaterials auffangen. Günstig ist es im Set zumindest zwei verschiedene Haftvermittler zur Verfügung zu stellen. Einer davon kann dann als Haftvermittler zwischen Zahn bzw. Zahnstumpf und Befestigungsmaterial und der andere Haftvermittler zwischen Befestigungsmaterial und Zahnfrontverblendungskörper eingesetzt werden. Als Haftvermittler sind z.B. die mit REF 2050 und REF 2051 bezeichneten Produkte der Firma Indigo-dental GmbH & Co. KG, in Pinneberg, Deutschland geeignet. Das Produkt REF 2050 kann z.B. als Haftvermittler zwischen Zahnstumpf und Befestigungsmaterial verwendet werden. Es enthält Methacrylierte Polyacrylsäure in einer Bis- GMA basierten Matrix. Das Produkt REF 2051 kann z.B. als Haftvermittler zwischen Befestigungsmaterial und Zahnfrontverblendungskörper verwendet werden. Es handelt sich dabei auch um ein Bis-GMA basiertes Methacrylat.

Das Ätzmittel z.B. in Form eines Ätzgels dient dazu, die durch den Bohrer des Zahnarztes verursachte Schmierschicht wegzuätzen und die Tubuli freizulegen. Geeignete Ätzmittel sind z.B. 15 -37%ige Phosphorsäure oder das Produkt REF 2052 der oben bereits genannten Firma. Es ist auch denkbar, bereits fertige Mischungen aus Ätzmittel und Haftvermittler einzusetzen. Generell kann es sich bei den Haftvermittlern um niedermolekulare Methacrylate handeln. Es können auch Monomere eingesetzt werden, welche ein hydrophiles und ein hydrophobes Ende haben, wie z.B. eine methacrylierte Polyacrylsäure.

Als zusammengesetztes Material für die Herstellung des Zahnfrontverblendungskörpers aber auch als Befestigungsmaterial kann z.B. das unter der Bezeichnung REF 2061erhältliche Produkt der Firma Indigodental GmbH & Co. KG, in Pinneberg, Deutschland verwendet werden. Dieses Produkt enthält Triethylenglykoldimethacrylat, Urethandimethacrylat, Bis-GMA und ethoxyliertes Bisphenol A Dimethacrylat. Als organisches Bindemittel kann z.B. das bereits bezüglich der Haftvermittler genannte Produkt REF 2050 dieser Firma verwendet werden. Als Farbstoffe, insbesondere zum Einfärben des Befestigungsmaterials können z.B. organische Pigmente und/oder der anorganische Weißmacher TiO2 verwendet werden.

Weitere Einzelheiten und Merkmale bevorzugter Ausgestaltungsformen der Erfindung ergeben sich aus der nachfolgenden Figurenbeschreibung. Dabei zeigen:
Fig. 1 bis 3 verschiedene erfindungsgemäße Zahnfrontverblendungskörper für Zähne des Oberkiefers in Frontalansicht;
Fig. 4 bis 6 Längsschnitte durch den jeweils darüber angeordneten Zahnfrontverblendungskörper gemäß der Fig. 1 bis 3;
Fig. 7 bis 9 verschiedene erfindungsgemäße Zahnfrontverblendungskörper für Zähne des Unterkiefers;
Fig. 10 bis 12 Längsschnitte durch den jeweils darüber angeordneten Zahnfrontverblendungskörper gemäß der Fig. 7 bis 9;
Fig. 13 eine schematisierte Explosionsdarstellung zum Anbringen des Zahnfrontverblendungskörpers an einem Zahnstumpf;
Fig. 14 eine schematisierte Seitenansicht des am Zahnstumpf angebrachten Zahnfrontverblendungskörpers;
Fig. 15 eine schematisierte Mikroaufnahme des zusammengesetzten Materials des Zahnfrontverblendungskörpers und
Fig. 16 eine erfindungsgemäße Ausführungsform eines Sets.

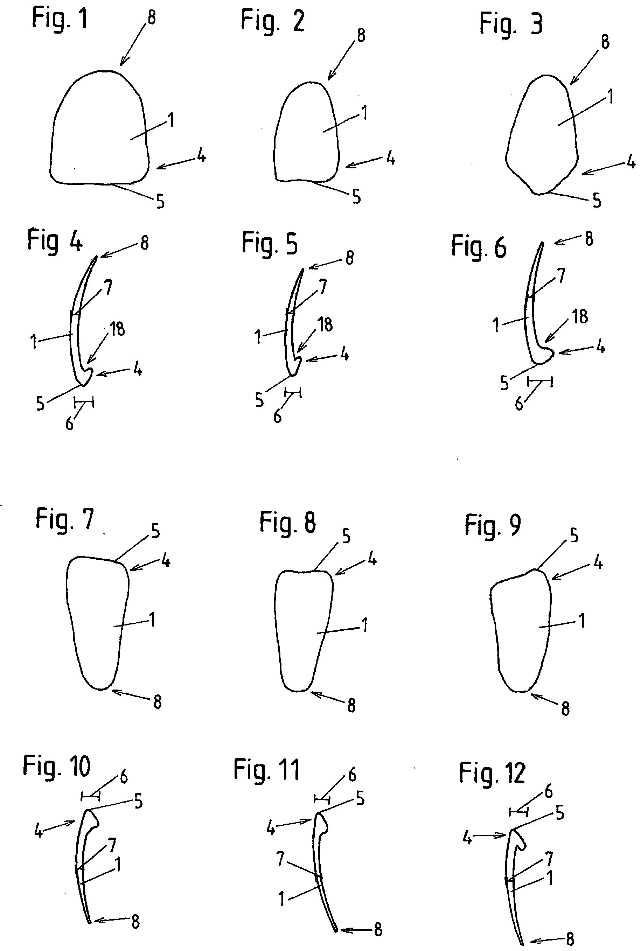

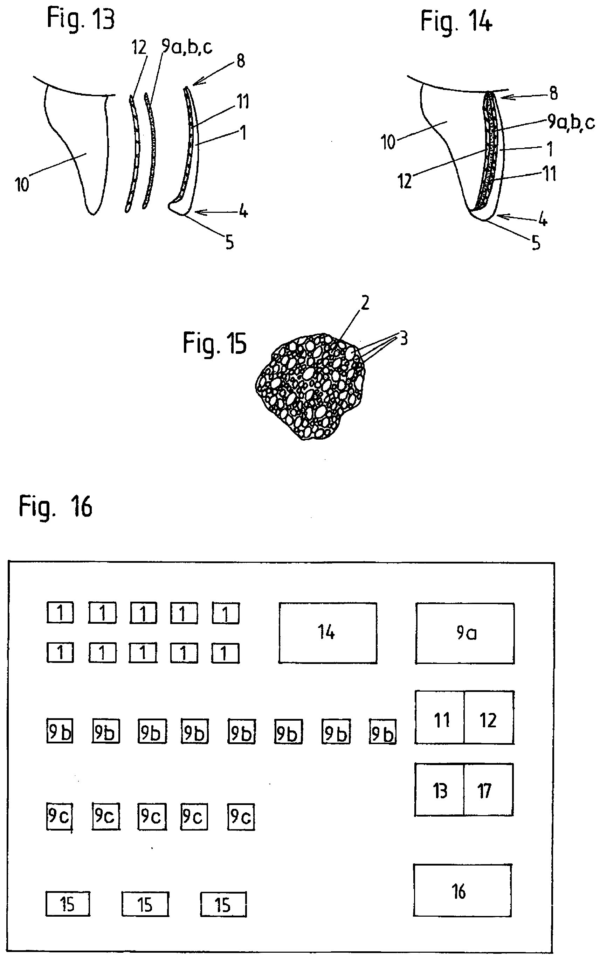

Die Fig. 1 bis 3 zeigen verschiedene Zahnfrontverblendungskörper 1 für verschiedene Zähne des Oberkiefers. Die Fig. 7 bis 9 zeigen verschiedene Zahnfrontverblendungskörper 1 für verschiedene Zähne des Unterkiefers. Untersuchungen haben gezeigt, dass es möglich ist, die bei unterschiedlichsten Patienten vorkommenden Zahnformen so zu generalisieren, dass man mit relativ wenigen verschieden vorgeformten Rohlingen der Zahnfrontverblendungskörper 1 fast alle natürlich vorkommenden Zahnformen abdecken kann, wenn der Zahnarzt vor Ort den richtigen Rohling des Zahnfrontverblendungskörpers 1 auswählt und an die tatsächlich gegebene Zahnform des Patienten anpasst, was bei erfindungsgemäßen Zahnfrontverblendungskörpern 1 im Gegensatz zum Stand der Technik möglich ist. Man kommt daher mit einer relativ geringen Anzahl an Sätzen von Rohlingen aus. Diese können z.B. in drei unterschiedlichen Größenstufen für den Oberkiefer und zwei unterschiedlichen Größenstufen für den Unterkiefer angeboten werden. Die Fig. 4 bis 6 und 10 bis 12 zeigen jeweils einen Längsschnitt durch den Zahnfrontverblendungskörper 1 der darüber angeordneten Figur. Jeder der dargestellten Zahnfrontverblendungskörper 1 weist einen Endbereich 4 im Bereich der Schneidkante 5 auf. Die Schneidkante 5 tritt beim Kauen jeweils direkt mit dem zu zerkauenden Nahrungsmittel in Kontakt um es zu zerteilen. Wie insbesondere in den Längsschnitten gemäß der Fig. 4 bis 6 und 10 bis 12 zu sehen ist, ist bei dem gezeigten Ausführungsbeispiel vorgesehen, dass der Zahnfrontverblendungskörper 1 im Endbereich 4 der Schneidkante 5 seine größte Dicke 6 aufweist. Die Dicke 6 liegt günstigerweise zwischen 1mm und 1,3mm. Im Endbereich 4 sind die dargestellten Zahnfrontverblendungskörper 1 im Längsschnitt gesehen zumindest auf der Rückseite haken- bzw. stufenförmig ausgebildet, sodass die Schneidkante 5, wenn der Zahnfrontverblendungskörper 1 am Zahnstumpf 10 angebracht ist, die gesamte Schneidkante des so wieder hergestellten Zahnes bildet. Der Zahnstumpf 10 liegt, wie in Fig. 14 zu sehen, dann form-schlüssig am freien Ende 18 des im Längsschnitt gesehen hakenförmig bzw. stufenförmig ausgebildeten Endbereichs 4 an. Außerhalb des Endbereichs 4 weist der Zahnfrontverblendungskörper 1 eine wesentlich geringere Dicke 7 auf. Diese erreicht günstigerweise maximal 0,6mm. Am der Schneidkante 5 gegenüberliegenden Ende 8 laufen die gezeigten Ausführungsbeispiele von Zahnfrontverblendungskörpern 1 flach aus.

Insgesamt ergibt sich eine zumindest bereichsweise schalenförmig gewölbte Ausformung der Zahnfrontverblendungskörper 1.

Fig. 13 zeigt grob schematisiert einen bereits fertig präparierten und angeätzten Zahn-stumpf 10. Auf diesen wird zunächst ein erster Haftvermittler 12 aufgebracht, welcher einer innigen Verbindung zwischen Zahnstumpf 10 und Befestigungsmaterial 9a, b oder c dient. Günstigerweise handelt es sich bei dem Befestigungsmaterial um ein pastöses Material. Günstigerweise weist das Befestigungsmaterial dasselbe zusammengesetzte Material, also sowohl organisches Bindemittel 2 als auch Feststoffpartikel 3 auf, aus dem auch der Zahnfrontverblendungskörper 1 hergestellt wird, allerdings eben noch in pastöser Konsistenz. Zusätzlich ist das Befestigungsmaterial allerdings noch eingefärbt, um im Zusammenwirken mit der Transluzenz des Zahnfrontverblendungskörpers im fertig am Zahn befestigten Zustand die gewünschte Färbung zu ergeben. Für die großflächige Befestigung ist vor-zugsweise vorgesehen, dass das Befestigungsmaterial 9b und 9c sowohl organisches Bindemittel 2 als auch Feststoffpartikel 3 aufweist. Dieses Material ist allerdings relativ breiig bzw. pastös. Um Bereiche mit sehr feingliedriger Oberflächenstruktur bearbeiten zu können, kann anstelle dieses Befestigungsmaterials 9b und 9c auch ein Befestigungsmaterial 9a verwendet werden, welches abgesehen von der Einfärbung ausschließlich organisches Bindemittel 2 oder zumindest einen geringeren Anteil an Feststoffpartikeln 3, von z.B. 60 bis 70 Vol-% als Füllstoff aufweist. Dieses Befestigungsmaterial 9a kann dasselbe organische Bindemittel, vorzugsweise auf Methacrylatbasis, und auch dieselbe Art und Mischung von Feststoffpartikeln, aber eben in geringerer Konzentration, aufweisen, welche auch das zusammengesetzte Material des fertigen Zahnfrontverblendungskörpers 1 aufweist. Zwischen dem Befestigungsmaterial 9a, b oder c und dem Zahnfrontverblendungskörper 1 wird günstigerweise wiederum ein Haftvermittler 11 angebracht. Besonders bevorzugt handelt es sich dabei um einen anderen Haftvermittler 11 als den Haftvermittler 12. Geeignete Beispiele von Haftvermittlern wurden eingangs genannt.

Fig. 14 zeigt den fertigen Zustand, in dem der Zahnfrontverblendungskörper 1 mittels des Befestigungsmaterials 9a, b oder c am Zahnstumpf 10 unter Zwischenschaltung der Haftvermittler 11 und 12 befestigt ist. Die im Befestigungsmaterial 9a, b oder c vorhandenen Farbpigmente scheinen durch das transluzente Material des Zahnfrontverblendungskörpers 1 hindurch und geben so, ähnlich wie beim natürlichen Zahn, die gewünschte Färbung.

Fig. 15 ist eine schematisierte Vergrößerung des zusammengesetzten Materials des Zahnfrontverblendungskörpers 1. Die verschiedenen Feststoffpartikel 3 sind mittels des organischen Bindemittels 2 innig miteinander verbunden. Als organisches Bindemittel kommen vorzugsweise methacrylatbasierte Harze zum Einsatz. Ein konkretes Beispiel eines geeigneten organischen Bindemittels 2 ist weiter oben genannt. Die Feststoffpartikel 3 bestehen bevorzugt aus einer Glasmischung mit Feststoffpartikeln 3 unterschiedlicher Korngröße. Hierdurch wird eine sehr dichte Packung und damit eine hohe Stabilität und Abrasionsfestigkeit des zusammengesetzten Materials erreicht. Günstigerweise beträgt der Volumenanteil der Feststoffpartikel 3 im Zahnfrontverblendungskörper 1 zumindest 75%, vor-zugsweise zumindest 82%, am zusammengesetzten Material. Um auch die Lücken zwischen den relativ großen Körnern möglichst vollständig ausfüllen zu können, sehen besonders bevorzugte Ausgestaltungsformen der Erfindung vor, dass die Korngrößen zumindest eines Teils der Feststoffpartikel 3 zwischen 0,01 µm und 3 µm liegen.

Fig. 16 zeigt schematisiert ein Set mit mehreren erfindungsgemäßen Zahnfrontverblendungskörpern 1 in verschiedenen Größen und diversen anderen Hilfsmitteln, um dem Zahnarzt die Anpassung und das Anbringen des Zahnfrontverblendungskörpers 1 am Zahnstumpf 10 innerhalb eines einzigen Behandlungstermins zu ermöglichen. Das Set gemäß Fig. 16 weist zunächst verschiedene Sätze von verschieden großen und verschieden ausgeformten Zahnfrontverblendungskörpern 1 auf. Im gezeigten Ausführungsbeispiel gibt es jeweils einen Satz von Zahnfrontverblendungskörpern 1 für die Zähne des Oberkiefers in drei verschiedenen Größen. Für den Unterkiefer sind im gezeigten Set Sätze in zwei unterschiedlichen Größen vorgesehen. Die Zahnfrontverblendungskörper 1 sind dabei in verschiedenen Grundformen aufgebaut. Der Größenunterschied zwischen den kleinen, mittleren und größeren Ausgestaltungsformen beträgt vorzugsweise jeweils 10%. Der Zahnarzt kann aus diesem Angebot von Zahnfrontverblendungskörpern 1 den für den jeweiligen Zahn des Patienten am besten passenden aussuchen und die Feinanpassung dann mittels entsprechendem Zuschleifen vor Ort durchführen. Um dem Zahnarzt das Auswählen des richtigen Zahnfrontverblendungskörpers 1 zu erleichtern, weist das Set gemäß Fig. 16 verschiedene Formschablonen 15 auf, mit denen in einfacher Art und Weise zunächst der optimal vorgeformte Zahnfrontverblendungskörper 1 ausgewählt werden kann. Neben den Zahnfrontverblendungskörpern 1 weist das Set gemäß Fig. 16 noch verschiedenste Befestigungsmaterialien 9a, b und c auf. Bei 9a handelt es sich um, in verschiedene Spritzen abgefülltes, verschieden eingefärbtes organisches Bindemittel 2, gegebenenfalls mit einem geringen Anteil an Feststoffpartikeln 3, vorzugsweise in der oben genannten Konzentration. Das Befestigungsmaterial 9a wird dann eingesetzt, wenn es darum geht, sehr kleine Hohlräume mit Befestigungsmaterial zu füllen. Das Befestigungs-material 9a kann abgesehen von den Farbpigmenten und gegebenenfalls weiteren Zusätzen im Wesentlichen dem organischen Bindemittel 2, welches das zusammengesetzte Material der Zahnfrontverblendungskörper 1 dieses Sets aufweist, gegebenenfalls zusätzlich mit dem genannten geringen Anteil an Feststoffpartikeln 3, entsprechen. Bei den Befestigungsmaterialien 9b und 9c handelt es sich um verschieden eingefärbte pastöse Befestigungsmaterialien. Diese weisen günstigerweise das zusammengesetzte Material der Zahnfrontverblendungskörper 1 in noch nicht ausgehärteter Konsistenz auf. Die Befestigungsmaterialien 9b und 9c umfassen somit sowohl das organische Bindemittel 2 als auch die Feststoffpartikel 3. Der Zahnarzt kann mit Hilfe des Farbschlüssels 14 das geeignet eingefärbte Befestigungsmaterial 9a, b oder c auswählen und in der in Fig. 13 angedeuteten Art und Weise zum Befestigen des Zahnfrontverblendungskörpers 1 am Zahnstumpf 10 verwenden. Im gezeigten Ausführungsbeispiel sind die verschiedenen Befestigungsmaterialien 9c für Fälle vorgesehen, in denen am Zahnstumpf 10 noch natürlicher Zahnschmelz vorhanden ist. Die hier gewählten Farben sind daher sehr hell. Die verschiedenen Befestigungsmaterialien 9b sind für den Fall vorgesehen, dass der Zahnschmelz vom Zahnstumpf 10 vollständig entfernt ist und mit kräftigeren Farben gearbeitet werden muss, damit das gewünschte Ergebnis erreicht wird. Das Set gemäß Fig. 16 umfasst im gezeigten Ausführungsbeispiel auch Applikationsnadeln 16, welche dazu dienen, das Befestigungsmaterial 9a, b und/oder c auf den Zahnstumpf 10 aufzubringen. 11 und 12 sind zwei verschiedene Haftvermittler die, wie in Fig. 13 schematisiert gezeigt, verwendet werden können. Weiters umfasst das Set gemäß Fig. 16 ein Ätzmittel 13, welches dazu dient, vor Aufbringung des jeweiligen Haftvermittlers 11 bzw. 12 die durch die vorherige Bearbeitung des Zahnstumpfes 10 verursachte Schmierschicht wegzuätzen und die Tubuli bzw. Zahnstümpfe bzw. Zähne freizulegen. Im gezeigten Ausführungsbeispiel ist zusätzlich auch noch ein Mittel 17 vorgesehen, welches eine Kombination aus einem Ätzmittel und einem Haftvermittler ist. Bevorzugte Ausgestaltungsformen von entsprechenden Sets werden in entsprechenden Behältern angeboten. Die Aufteilung der Fächer und der Inhalt sind in Fig. 16 natürlich nur beispielhaft und schematisiert dargestellt.

Ein Verfahren zur Herstellung erfindungsgemäßer Zahnfrontverblendungskörper 1 aus dem z.B. im Handel erhältlichen, zusammengesetzen und eingangs genannten Material kann folgende Schritte vorsehen:
Zunächst wird das zusammengesetzten Material bzw. Composite, umfassend das organische Bindemittel 2 und die Feststoffpartikel 3, unter Vakuum in Kartuschen gefüllt, auf ca. 60° Celsius erwärmt und mit 80 bis 100bar Druck in die entsprechend einseitig transparent ausgeführten Zahnformen injiziert. Die Zahnformen können aus Glas und Chromstahl hergestellt sein. Die Aushärtung des zusammengesetzten Materials kann dann mit einem geeigneten LED-Licht und einer Belichtungszeit von z.B. 60 Sekunden erfolgen. Ein geeignetes LED-Licht ist z.B. charakterisiert durch eine Wellenlänge von 450 Nanometern (nm) bis 480 nm. Danach können die Zahnfrontverblendungskörper 1 in einem Vakuumdruckluftofen über einen Zeitraum von z.B. zehn Minuten auf z.B. 100° erwärmt und thermisch vergütet werden. Im Anschluss daran werden die Rohlinge der Zahnfrontverblendungskörper 1 aus den Zahnformen herausgenommen. Anschließend kann ein Zuschneiden oder Nachbearbeiten der Form erfolgen.

### Legende zu den Hinweisziffern:

- 1: Zahnfrontverblendungskörper
- 2: Bindemittel
- 3: Feststoffpartikel
- 4: Endbereich
- 5: Schneidekante
- 6: Dicke
- 7: Dicke
- 8: Ende
- 9a, b, c: Befestigungsmaterial
- 10: Zahnstumpf
- 11: Haftvermittler
- 12: Haftvermittler
- 13: Ätzmittel
- 14: Farbschlüssel
- 15: Formschablonen
- 16: Applikationsnadel
- 17: Ätzmittel und Haftvermittler
- 18: freies Ende

## Patentansprüche

1. Als Festkörper ausgebildeter Zahnfrontverblendungskörper (1) zur Aufsetzung auf einen bearbeiteten Zahn, um eine Frontfläche des Zahnes zu verblenden, bestehend aus einem zusammengesetztem Material, wobei das zusammengesetzte Material zumindest ein organisches Bindemittel (2), vorzugsweise mit Methacrylat, und, vorzugsweise anorganische, Feststoffpartikel (3) als Füllstoff enthält, wobei der Zahnfrontverblendungskörper an einem seiner Endbereiche (4) im Längsschnitt durch den Zahnfrontverblendungskörper haken- bzw. stufenförmig ausgebildet ist mit einer Schneidkante (5) in diesem Endbereich (4), wobei der Zahnfrontverblendungskörper (1) ausserhalb des Endbereichs (4) mit der Schneidkante (5) maximal eine Dicke (7) von 0,6 mm aufweist und/oder an seinem der Schneidkante gegenüberliegenden Ende (8) flach ausläuft.

2. Zahnfrontverblendungskörper (1) nach Anspruch 1, **dadurch gekennzeichnet, dass** er zumindest bereichsweise schalenförmig gewölbt ausgebildet ist.

3. Zahnfrontverblendungskörper (1) nach Anspruch 1 oder 2, **dadurch gekennzeichnet, dass** die Feststoffpartikel (3) des Füllstoffs Glas, vorzugsweise eine Mischung verschiedener Glasarten, aufweisen oder daraus bestehen, und/oder dass der Füllstoff Feststoffpartikel (3) mit voneinander verschiedenen Korngrössen, insbesondere mit Korngrössen zwischen 0,01um und 3µm, aufweist.

4. Zahnfrontverblendungskörper (1) nach einem der Ansprüche 1 bis 3, **dadurch gekennzeichnet, dass** die Feststoffpartikel (3) des Füllstoffs einen Anteil von zumindest 75 Vol-%, vorzugsweise von zumindest 82 Vol-%, am zusammengesetzten Material des Zahnfrontverblendungskörper (1) haben.

5. Zahnfrontverblendungskörper (1) nach einem der Ansprüche 1 bis 4, **dadurch gekennzeichnet, dass** er transluzent ist, vorzugsweise eine Transluzenz von 28% bis 39% aufweist, und/oder einen L-Wert von 59 bis 69 aufweist und/oder einen a-Wert von -0,35 bis -3,2 aufweist und/oder einen b-Wert von -0,4 bis -6,95 aufweist.

6. Zahnfrontverblendungskörper (1) nach einem der Ansprüche 1 bis 5, **dadurch gekennzeichnet, dass** die Schneidkante (5) eine Dicke (6) von 1 mm bis 1,3 mm aufweist.

7. Set mit mehreren, insbesondere verschieden grossen und/oder verschieden geformten, Zahnfrontverblendungskörper (1) nach einem der Ansprüche 1 bis 6, wobei das Set zusätzlich zumindest ein, vorzugsweise flüssiges oder pastöses, Befestigungsmaterial (9a, 9b, 9c) und mehrere verschiedene Farben zum Einfärben des Befestigungsmaterials (9a, 9b, 9c) und/oder mehrere verschiedenfarbige, vorzugsweise flüssige oder pastöse, Befestigungsmaterialien (9a, 9b, 9c) aufweist, wobei das Befestigungsmaterial (9a, 9b, 9c) bzw. die Befestigungsmaterialien (9a, 9b, 9c) zum Befestigen, vorzugsweise Ankleben, des Zahnfrontverblendungskörper (1) an einem Zahnstumpf geeignet (10) ist bzw. sind.

8. Set nach Anspruch 7, **dadurch gekennzeichnet, dass** das Befestigungsmaterial (9) dasselbe zusammengesetzte Material oder dasselbe organische Bindemittel enthält wie die Zahnfrontverblendungskörper (1).

9. Set nach Anspruch 7 oder 8, **dadurch gekennzeichnet, dass** das Set zumindest einen Haftvermittler (11, 12), vorzugsweise zumindest zwei verschiedene Haftvermittler (11, 12), und/oder zumindest ein Ätzmittel (13) aufweist.

## Claims

1. Tooth front veneer (1) configured as a solid body for placing onto a machined tooth so as to screen a front face of the tooth, composed of a material composition, wherein the material composition contains at least one organic binding agent (2), preferably comprising methacrylate, and preferably inorganic solids particles (3) as a filler material, wherein the tooth front veneer on one of the end regions (4) thereof, in the longitudinal section through the tooth front veneer, is configured so as to be hook-shaped or step-shaped, respectively, having a cutting edge (5) in said end region (4), wherein the tooth front veneer (1) outside the end region (4) having the cutting edge (5) has a maximum thickness (7) of 0.6 mm and/or at the end (8) of said front tooth veneer (1) that is opposite the cutting edge peters out in a flat manner.

2. Tooth front veneer (1) according to Claim 1, **characterized in that** said tooth front veneer (1) at least in regions is curved in a shell-like manner.

3. Tooth front veneer (1) according to Claim 1 or 2, **characterized in that** the solids particles (3) of the filler material comprise or are composed of glass, preferably a mixture of different glass types, and/or **in that** the filler material comprises solids particles (3) having mutually dissimilar grain sizes, in particular having grain sizes between 0.01 µm and 3 µm.

4. Tooth front veneer (1) according to one of Claims 1 to 3, **characterized in that** the solids particles (3) of the filler material account for a proportion of at least 75% by volume, preferably of at least 82% by volume, of the material composition of the tooth front veneer (1).

5. Tooth front veneer (1) according to one of Claims 1 to 4, **characterized in that** said tooth front veneer is translucent, preferably having a translucence of 28% to 39%, and/or has an L-value of 59 to 69, and/or has an α-value of -0.35 to - 3.2, and/or has a b-value of -0.4 to -6.95.

6. Tooth front veneer (1) according to one of Claims 1 to 5, **characterized in that** the cutting edge (5) has a thickness (6) of 1 mm to 1.3 mm.

7. Set having a plurality of tooth front veneers (1), according to one of Claims 1 to 6, in particular of different sizes and/or shapes, wherein the set additionally comprises at least one, preferably liquid or pasty, fastening material (9a, 9b, 9c) and a plurality of different colours for colouring the fastening material (9a, 9b, 9c), and/or a plurality of, preferably liquid or pasty, fastening materials (9a, 9b, 9c) of different colours, wherein the fastening material (9a, 9b, 9c) or the fastening materials (9a, 9b, 9c), respectively, is/are suitable for fastening, preferably adhesively bonding, the front tooth veneer (1) to a tooth stump (10).

8. Set according to Claim 7, **characterized in that** the fastening material (9) contains the same material composition or the same organic binding agent as the tooth front veneer (1).

9. Set according to Claim 7 or 8, **characterized in that** the set comprises at least one adhesion promoter (11, 12), preferably at least two different adhesion promoters (11, 12), and/or at least one caustic agent (13).

## Revendications

1. Corps de facette avant de dent (1) réalisé sous forme de corps solide à poser sur une dent traitée, afin de recouvrir une face avant de la dent, constitué d'un matériau composé, dans lequel le matériau composé contient au moins un liant organique (2), de préférence avec du méthacrylate, et des particules de matière solide (3), de préférence inorganiques, comme matière de remplissage, dans lequel le corps de facette avant de dent est réalisé à une de ses régions d'extrémité (4) en coupe longitudinale à travers le corps de facette avant de dent sous forme de crochet ou sous forme étagée avec une arête de coupe (5) dans cette région d'extrémité (4), dans lequel le corps de facette avant de dent (1) présente en dehors de la région d'extrémité (4) avec l'arête de coupe (5) au maximum une épaisseur (7) de 0,6 mm et/ou se termine à plat à son extrémité (8) opposée à l'arête de coupe.

2. Corps de facette avant de dent (1) selon la revendication 1, **caractérisé en ce qu'**il est réalisé au moins localement sous forme bombée en coquille.

3. Corps de facette avant de dent (1) selon une revendication 1 ou 2, **caractérisé en ce que** les particules de matière solide (3) de la matière de remplissage présentent du verre, de préférence un mélange de différents types de verre, ou en sont constituées, et/ou **en ce que** la matière de remplissage présente des particules de matière solide (3) ayant des tailles de grains différentes les unes des autres, en particulier des tailles de grains entre 0,01 µm et 3 µm.

4. Corps de facette avant de dent (1) selon l'une quelconque des revendications 1 à 3, **caractérisé en ce que** les particules de matière solide (3) de la matière de remplissage représentent une part d'au moins 75 % en volume, de préférence d'au moins 82 % en volume, du matériau composé du corps de facette avant de dent (1).

5. Corps de facette avant de dent (1) selon l'une quelconque des revendications 1 à 4, **caractérisé en ce qu'**il est translucide, qu'il présente de préférence une translucidité de 28 % à 39 %, et/ou qu'il présente une valeur L de 59 à 69 et/ou qu'il présente une valeur a de -0,35 à -3,2 et/ou qu'il présente une valeur b de - 0,4 à -6,95.

6. Corps de facette avant de dent (1) selon l'une quelconque des revendications 1 à 5, **caractérisé en ce que** l'arête de coupe (5) présente une épaisseur (6) de 1 mm à 1,3 mm.

7. Ensemble de plusieurs corps de facette avant de dent (1) selon l'une quelconque des revendications 1 à 6, en particulier de tailles différentes et/ou de formes différentes, dans lequel l'ensemble présente en outre au moins un matériau de fixation (9a, 9b, 9c), de préférence liquide ou pâteux, et plusieurs couleurs différentes pour colorer le matériau de fixation (9a, 9b, 9c) et/ou plusieurs matériaux de fixation de couleurs différentes (9a, 9b, 9c), de préférence liquides ou pâteux, dans lequel le matériau de fixation (9a, 9b, 9c) ou les matériaux de fixation (9a, 9b, 9c) est/sont approprié (s) pour la fixation, de préférence le collage, du corps de facette avant de dent (1) sur un moignon de dent (10).

8. Ensemble selon la revendication 7, **caractérisé en ce que** le matériau de fixation (9) contient le même matériau composé ou le même liant organique que le corps de facette avant de dent (1).

9. Ensemble selon une revendication 7 ou 8, **caractérisé en ce que** l'ensemble présente au moins un agent adhésif (11, 12), de préférence au moins deux agents adhésifs différents (11, 12), et/ou au moins un agent de gravure (13).
